# EUROPEAN PATENT SPECIFICATION

(11) **EP 3 646 805 B1**
(45) Date of publication and mention of the grant of the patent: **17.08.2022**
(21) Application number: 19206836.9
(22) Date of filing: 04.11.2019
(51) Int. Cl.: A61B 17/17, A61B 17/88, A61B 17/56, A61B 17/80

(54) **DEVICE FOR FIXATING ORTHOPEDIC INJURY**
VORRICHTUNG ZUM FIXIEREN VON ORTHOPÄDISCHEN VERLETZUNGEN
DISPOSITIF DE FIXATION D'UNE LÉSION ORTHOPÉDIQUE

(30) Priority: 01.11.2018 US 201862754342 P
(43) Date of publication of application: 06.05.2020
(73) Proprietor: Howmedica Osteonics Corporation, Mahwah, New Jersey 07430 (US)
(72) Inventor: BRIGIDO, Stephen A., Bethlehem, PA Pennsylvania 18015 (US); HYER, Christopher, Columbus, OH Ohio 43220 (US); BERLET, Gregory, Westerville, OH Ohio 43082 (US); PENNER, Murray, Vancouver, British Columbia 3140 (CA); SANDER, Jeffrey Duncan, Liberty Township, OH Ohio 45011 (US)
(74) Representative: Röthinger, Rainer

(56) References cited:
- DE-A1-102008 004 922
- FR-A1- 2 764 183
- US-A1- 2008 154 384
- US-A1- 2009 210 011
- US-A1- 2014 236 305
- US-A1- 2017 042 599
- US-A1- 2017 105 775
- US-A1- 2018 110 530

## Description

### BACKGROUND OF THE INVENTION

The present invention relates generally to an alignment and fixation device for use during orthopedic surgeries, and more specifically surgeries of the foot and/or ankle, for example, Lisfranc procedures or a Lapidus bunionectomy.

Together, the foot and ankle are composed of 26 bones and 33 joints, along with more than 100 muscles, tendons, and ligaments, all residing beneath a relatively slim covering of soft tissue and skin. Structurally, the foot has three main anatomical regions: the forefoot, the midfoot and the hindfoot. These regions work together with the ankle to provide the body with support, balance and mobility. A structural flaw or malfunction in any one region can manifest in injuries in other regions of the foot or areas of the body.

Foot and ankle injuries typically occur as a result of dropping a heavy object on the top of the foot, stepping on an uneven surface or falling with the foot in a twisted position. Foot and ankle injuries also commonly occur in athletes when the foot is bound and subjected to simultaneous impact and rotation.

A common midfoot injury is a Lisfranc injury. The Lisfranc joint complex is composed of the tarsometatarsal (TMT) joints and the proximal intermetatarsal and anterior intertarsal joint articulation. As a result of its position in the midfoot, the Lisfranc joint provides critical structural support and stability to the transverse arch of the midfoot. Typically, a Lisfranc injury displaces one or more of the metatarsal bones. Treatment of Lisfranc injuries are complicated by the fact that these injuries are often difficult to diagnose due to the range of clinical presentations. Even when properly diagnosed, achieving proper anatomical reduction is challenging due to the intricacies of the bony architecture within the Lisfranc joint.

Treatment typically requires open reduction and internal fixation. Open reduction refers to the surgical repair of a fracture or dislocation to properly realign the bones. Open reduction allows direct visualization and reduction of the fracture-dislocation and enables debridement of the commuted fracture fragments, soft tissue, and osteochondral debris that frequently exist as a result of the traumatic nature of the injury. Internal fixation refers to fixation of screws, plates or intramedullary bone nails and allows for the bone to heal.

Bunions are another common foot deformity. A bunion is a distortion of bones and joints of a person's foot and is associated with hallux abducto valgus, or the movement of the great toe laterally. This condition is often painful and debilitating. Bunions are typically caused by poor biomechanics of the foot and tight footwear, which may aggravate the condition.

A Lapidus bunionectomy is a surgical procedure to remove painful bunions, by reforming the metatarsal. Typically, a bunionectomy surgery involves correction of the foot by reconstructing bones and joints. While there are numerous ways to correct bunions, the most common is an osteotomy, and specifically a chevron osteotomy. In a chevron osteotomy, the bone is cut at the distal end. The cut is made in a V-shape near the distal metacarpal joint, which allows the entire toe to be moved laterally to the correct alignment. A small screw is then fixed to the joint to provide stability while the bone heals.

Studies have shown that achieving and maintaining anatomical reduction is critical for optimal outcomes when performing Lisfranc or Lapidus procedures. Due to the significant operative challenges in treating these foot deformations, it is desirable to use instruments which improve surgical efficiency and the accuracy with which the displaced bones are aligned and fixed.

Therefore, there is a need for an easy to use device that guarantees accurate alignment of fractured bones and allows for internal fixation of the same to facilitate proper healing.

US 2009/210011 A1 discloses an orthopedic plate configured for implantation at the mid-foot. The plate has a first radius along the x-axis in the y direction and a second radius along the y-axis in the y direction The plate has an ring-shaped footprint with two diagonal sets of opposing tabs. In each set of tabs, one tab includes a compression slot that extends and causes compression in a direction toward a screw hole in the opposing tab of the other set of tabs. The maximum length of the plate extends between the longer set of tabs, and an opening is included along this axis to allow viewing of the bone/bone fragments under the plate. The placement of the compression slots and the screw holes account for the progressively anterior placement of the cuneiforms and provides for compression in the medial direction. In a further embodiment, the plate is flat or includes a crease midway between the first and second tabs, which facilitates placement of the plate at the joint between the metatarsals and the cuneiforms.

US 2018/110530 A1 discloses an osteotomy system including an alignment device, at least one k-wire for insertion into the alignment device, and a cut guide with at least one hole for receiving at least one k-wire and a slot for receiving a saw blade.

US 2017/042599 A1 discloses a technique for correcting a bone deformity, such as a bunion, using a fulcrum. The technique involves inserting a fulcrum between a first metatarsal that is anatomically misaligned with respect to a second metatarsal. The technique further includes preparing an end of the first metatarsal and preparing an end of a medial cuneiform opposing the end of the first metatarsal. In addition, a distal portion of the first metatarsal is moved toward the second metatarsal in a transverse plane, thereby pivoting a proximal portion of the first metatarsal about the fulcrum and reducing an intermetatarsal angle between the first metatarsal and the second metatarsal.

FR 2 764 183 A1 discloses a surgical apparatus for correction of hallux valgus. The apparatus has a rectangular plate of rigid metal with the ends having two points to be inserted into holes formed in the internal cortical of the first metatarsal, and with the zones situated between the ends and the median zone of the plate have two holes to receive two bone screws. The screws are inserted in the holes formed in the internal and external cortical of the first metatarsal. At least one piercing drill has four holes with positions corresponding to those of the points and the holes of the plate. A series of wedges have different thicknesses corresponding to the different possible degrees of correction.

DE 10 2008 004922 A1 discloses a guidance provided for a boring tool, and for attaching at bones, where the tool is utilized for producing a hollow space. The tool exhibits passages for several wire shaped strands to be driven into bones. The strands engage behind the guidance with bendable end sections on a side that is turned away from the bones under form-fit connection of the guidance with the bones. The guidance exhibits a form of a sleeve for guiding the boring tool. The guidance is inserted on a core element.

US 2014/236305 A1 discloses a surgical guide for implanting a knee prosthesis, capable of allowing the intraoperative balancing of the knee ligaments, comprising: a first component intended for uniquely coupling with the distal epiphyseal end of a femur; a template rotatably mounted with respect to said first component and intended for guiding a marking operation of said distal epiphyseal end aimed to determine the position of a knee prosthesis to be implanted; and a second component intended for solidly coupling said template with a proximal epiphyseal end of a tibia corresponding to said femur.

US 2008/154384 A1 discloses an elbow prosthesis and method of implanting same including an aggregate prosthesis having a retaining system in conjunction with a set plate.

US 2017/105775 A1 discloses plates for patella fracture reduction that universally fit to a front surface or perimeter of a patella and include a plurality of attachment holes disposed therein for receiving a fastener. The plates include removable tab members.

### SUMMARY OF THE INVENTION

The invention is defined by the independent claims. Advantageous embodiments are provided in accordance with the dependent claims and the description.

In accordance with a first aspect of the present invention, an alignment and bone fixation device for use in orthopedic surgery of a foot and/or ankle bone is defined in claim 1.

In accordance with another aspect of the invention, a kit for orthopedic surgery of a foot and/or ankle bone is defined in claim 13.

### BRIEF DESCRIPTION OF THE DRAWINGS

FIG. 1A is top plan view of healthy foot bones.
FIG. 1B is top plan view of foot bones having a Lisfranc injury.
FIGS. 2A-2C are perspective views of an alignment and fixation device in accordance with an embodiment of the present invention.
FIG. 3 is a perspective view in a posterior-anterior direction of the alignment and fixation device of FIGS. 2A-2C.
FIG. 4A is a side plan view in an anterior-posterior direction of the alignment and fixation device of FIGS. 2A-2C.
FIG. 4B is a side plan view in a medial-lateral direction of the alignment and fixation device of FIGS. 2A-2C.
FIG. 4C is a side plan view in a posterior-anterior direction of the alignment and fixation device of FIGS. 2A-2C
FIG. 5 is a side plan view in a medial-lateral direction of the alignment and fixation device of FIGS. 2A-2C.
FIG. 6 is top plan view of a kit for treating a Lisfranc injury including the alignment and fixation device of FIGS. 2A-2C.
FIGS. 7-12 illustrate the repair of a Lisfranc injury using the alignment and fixation device of FIGS. 2A-2C.
FIG. 13 is a top plan view of a repaired Lisfranc injury depicting internal fixation screws.
FIG. 14 is a side plan view in a posterior-anterior direction of an alignment and fixation device in accordance with another embodiment of the present invention.
FIG. 15 shows a bottom side view of an embodiment of a Chip as described herein.
FIGS. 16A-16C: FIG. 16B shows a top side view of an embodiment of a Chip as described herein. FIG. 16A shows a left side view of the Chip of Fig. 16B. Fig. 16C shows a right-side view of the Chip of Fig. 16B.
FIG. 17 shows a cross-sectional view of the Chip of Fig. 16B, along the line A-A.
FIG. 18 shows a first perspective view of an embodiment of a Chip as described herein.
FIG. 19 shows a second perspective view of an embodiment of a Chip as described herein.
FIG. 20 shows a third perspective view of an embodiment of a Chip as described herein.
FIG. 21 shows a fourth perspective view of an embodiment of a Chip as described herein.

### DETAILED DESCRIPTION

In describing preferred embodiments of the disclosure, directional anatomical nomenclature will be referenced to describe spatial relationships between different portions of the device. This nomenclature is used for convenience only and is not intended to be limiting with respect to the scope of the invention. For example, when used in connection with the alignment and fixation device, the term "anterior" means towards the front part of the body or the face and the term "posterior" means towards the back of the body when the device is attached to the foot and/or ankle of the patient. The term "medial" means toward the midline of the body and the term "lateral" means away from the midline of the body. The term "term "superior" means towards the head of the patient and the term "inferior" means toward the sole of the foot. When referring to the fixation elements (e.g., screws) or tools used to secure the fixation elements or realign displaced bone, the term "proximal" means closer to the user, wherein as the term "distal" means further from the user.

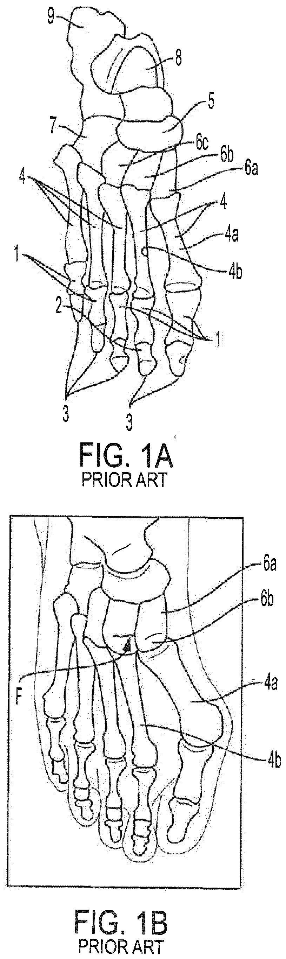

FIG. 1 illustrates the bone structure of a healthy right foot and is composed of three main regions: the forefoot, the midfoot, and the hindfoot. The forefoot is composed of the five toes, called phalanges and their connecting long bones, called metatarsals. Each toe, except the big toe, is composed of three phalanges (proximal phalange 1, middle phalange 2 and distal phalange 3) and two joints. Each of phalanges 1, 2, 3 are connected to metatarsals 4 by the metatarsal phalangeal joints at the ball of the foot. The big toe has two phalanges (distal phalange 3 and proximal phalange 1) and a joint called the interphalangeal joint. The big toe articulates with the head of the first metatarsal 4*a* and is called the first metatarsophalangeal joint (MTPJ). Underneath the first metatarsal 4*a* head are two tiny, round bones called sesamoids.

The midfoot has five irregularly shaped tarsal bones: navicular 5, medial cuneiform 6*a*, intermediate cuneiform 6*b*, lateral cuneiform 6*c*, and cuboid 7. The bones of the midfoot are connected to the forefoot and the hindfoot by muscles and the plantar fascia called arch ligament (not shown).

The hindfoot is composed of the talus 8 (lower ankle) and calcaneus 9 (heel). The superior end of talus 8 is connected to the tibia and fibula (not shown) of the lower leg and forms a hinge that allows the foot to move. Calcaneus 9 joins the talus 8 to form the subtalar joint.

Tarsometatarsal articulations are arthrodial joints in the foot, also known as the "Lisfranc joint." The Lisfranc joint is the medial articulation located at the intersection of the first *4a* and second metatarsals 4*b* and the medial 6*a* and intermediate 6*b* cuneiforms. The Lisfranc ligament is a large band of plantar collagenous tissue that spans the articulation of the medial cuneiform 6*a* and the base of the second metatarsal 4*b*. A Lisfranc injury is an injury to the Lisfranc joint and/or ligament and generally occurs as a result of direct or indirect trauma. The injury may be a dislocation such as a ligament injury, or a fracture and a dislocation. As shown in FIG. 1B, the fracture F usually occurs in the bones of the midfoot and the dislocation generally causes a separation of the joints between the forefoot and the midfoot and results in the misalignment of one or both of the first 4*a* and second metatarsals 4b relative to the tarsal bones.

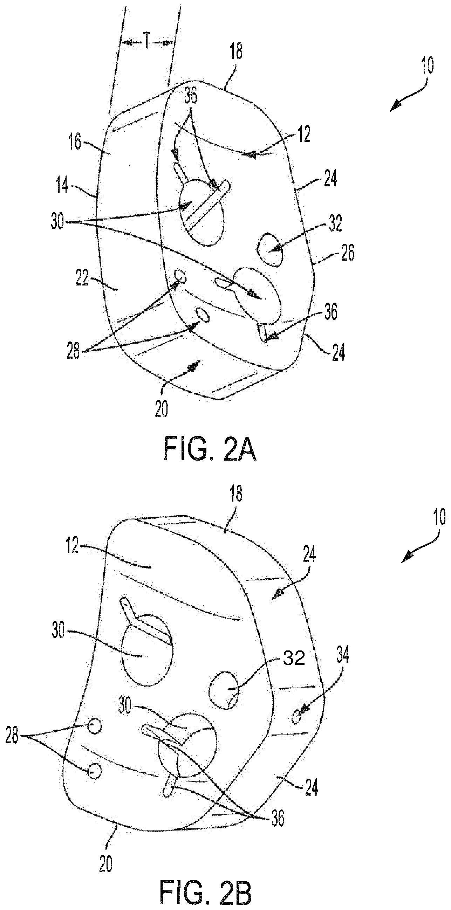

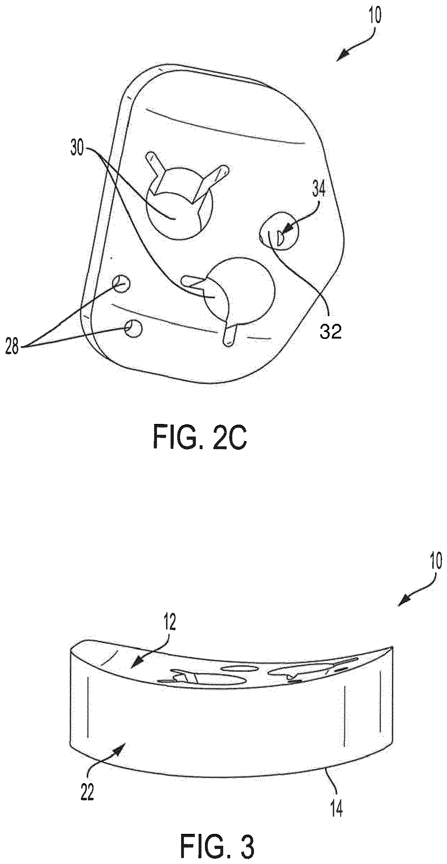

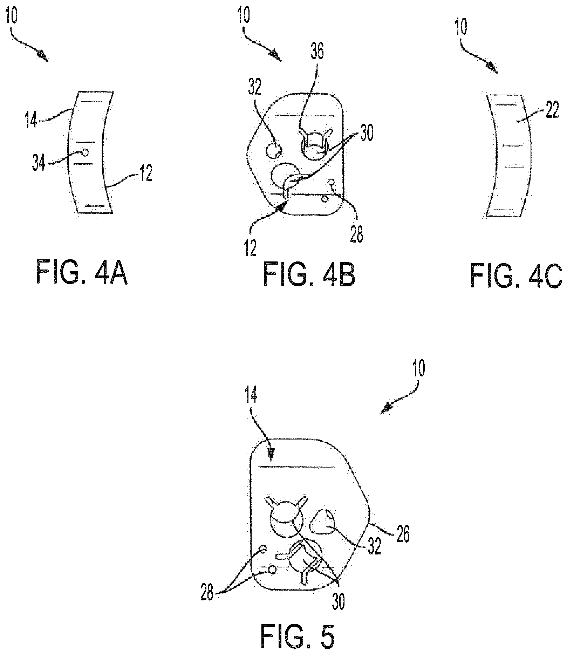

FIGS. 2A-5 illustrate an alignment and fixation device 10 for realigning and fixating displaced bone, for example, the metatarsal bones during a Lisfranc procedure. To facilitate understanding, fixation device 10 will be described hereinafter in the context of treating an injury to the Lisfranc joint. It will be appreciated, however, that device 10 may also be used to align and fixate fractures or dislocations of any foot/ankle bone or hand/wrist bone. For example, the alignment and fixation device may be used during surgeries to treat various injuries, including but not limited to a Lapidus bunionectomy, talar fractures, navicular fractures, cuneiform fractures, cuboid fractures, metatarsal base fractures, proximal and distal tibial fractures, distal radius fractures, fractures of the carpal bones, midfoot arthroplasty, hindfoot arthroplasty, and osteotomies of the hindfoot, midfoot and ankle.

Device 10 has a bone contacting surface 12, an opposing surface 14, a lateral surface 16 disposed between the bone contacting surface and the opposing surface and a longitudinal axis passing through the bone contacting surface and the opposing surface. The lateral surface 16 defines a thickness T of the device. In an exemplary embodiment, device 10 has a relatively small thickness T of between approximately 5-100 mm. In other embodiments, device 10 may have a thickness of greater than 100 mm. Device 10 can be made of any suitable material, but is preferably formed of a polymeric or thermoplastic material, for example, a glass-fiber reinforced grade of polyacrylamide (PARA) such as Ixef^{®} GS-1022 Polyarylamide (PARA). In the preferred embodiment, device 10 is intended to be a single use and disposable device. In this manner, device 10 need not be repeatedly sterilized, a process which can alter the shape of the device and thus reduce the accuracy with which the device can carry out a realignment procedure. Nevertheless, in other embodiments, device 10 may be formed from other materials suitable for orthopedic surgeries and capable of being sterilized on multiple occasions.

Device 10 is shaped generally as a pentagonal prism such that lateral surface 16 defines a superior surface 18, an inferior surface 20, a posterior surface 22, angled surfaces 24 and an anterior corner 26. As will be explained in further detail below, the pentagonal shape of the device assists a user in positioning device 10 in the proper location on the patient's midfoot. Device 10 may nevertheless be formed as any other shape, for example, a triangle or a rectangle. Each of the corners may be rounded to avoid pricks to the patient and/or the user.

In one exemplary embodiment, device 10 may have a length (e.g., the distance between superior surface 18 and inferior surface 20) of between approximately 15-200 mm, and a width (e.g., the distance between posterior surface 22 and anterior corner 26) of between approximately 15 mm and 100 mm. For example, the device may be approximately 32 mm in length and approximately 25 mm in width. The dimensions of device 10 may of course be modified based upon the size of the patient and the particular anatomical region upon which the device is intended to be positioned.

The bone contacting surface 12 may be concave in shape relative to a plane passing between the bone facing surface and the opposing surface in a direction orthogonal to the longitudinal axis. Bone contacting surface 12 is thus adapted to be positioned on a medial side of a patient's midfoot, and specifically, on the medial cuneiform 6a. The bone contacting surface 12, however, may be alternatively shaped and/or curved in accordance with the anatomical region upon which the device is intended to be positioned.

The opposing surface 14 may be convex in shape relative to the plane passing through the bone facing surface and the opposing surface in a direction orthogonal to the longitudinal axis. The convex shape of opposing surface 16 is ergonomically shaped and allows the device to be easily gripped during surgery.

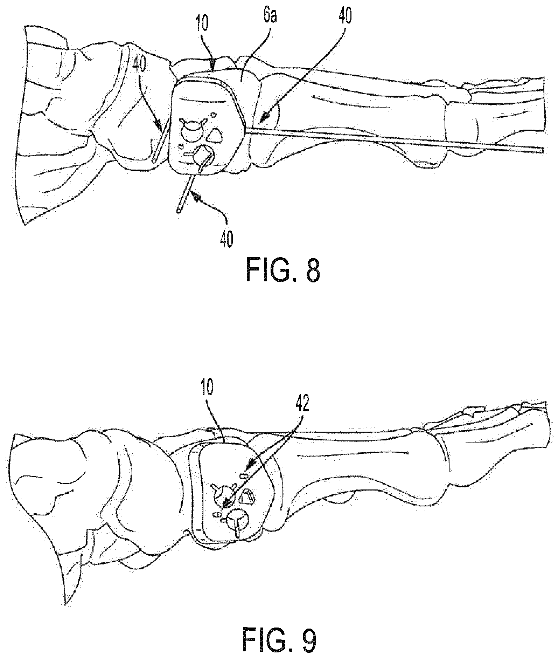

The body of device 10 defines at least one pin fixation borehole 28, at least one bone fastening borehole 30, an adjustment tool borehole 32 and an alignment index for positioning the body of the device relative to bone. Pin fixation borehole 28, bone fastening borehole 30 and adjustment tool borehole 32 extend completely through the body of the device from bone contacting surface 12 to opposing surface 14. In a preferred embodiment, device 10 includes at least two pin fixation boreholes 28 for receiving a pin and securing the device to the foot of a patient and two bone fastening boreholes 30 for guiding a bone fastener into bone. The diameter of each pin fixation borehole 28 may be between approximately 1.6-2.0 mm such that each pin fixation borehole is sized to receive a pin, such as a k-wire, having a diameter of approximately 1.6 mm or approximately 2.0 mm. Pin fixation boreholes 28 may be positioned at any location on the device but are preferably spaced a distance from bone fastening boreholes 30 and adjustment tool borehole 32 so as to not interfere with the bone fasteners or the adjustment tool. By way of example, pin fixation boreholes 28 may be disposed relatively close to one another, for example, adjacent the inferior and posterior sides of device 10, as shown in FIGS. 2A-5. Alternatively, pin fixation boreholes 28 may be positioned substantially on opposite sides of the device as shown in FIGS. 8-12.

A first bone fastening borehole 30 may be positioned at approximately the midline of the device while a second bone fastening borehole 30 may be inferiorly positioned relative to the first bone fastening borehole. For this reason, the first bone fastening borehole is sometimes referred to herein as the superior bone fastening borehole and the second bone fastening borehole is sometimes referred to herein as the inferior bone fastening borehole. Superior bone fastening borehole 30 may be angled in a superior-inferior direction and in a posterior-anterior direction relative to the longitudinal axis. In this manner, when device 10 is secured to the medial cuneiform 6*a*, the superior bone fastening borehole is adapted to guide a bone fastener, such as a bone screw, through the medial cuneiform 6*a*, the intermediate cuneiform *6b* and the lateral cuneiform 6*c*. Superior bone fastening borehole 30 may, for example, be angled in the superior-inferior direction at approximately 20 degrees relative to the longitudinal axis of the device, and angled in the posterior-anterior direction at approximately 2.5 degrees. The relatively small posterior-anterior angle ensures that the screw path is biased away from the posterior aspect of the medial cuneiform to prevent breakthrough.

The second or inferior bone fastening borehole 30 may be angled in an inferior-superior direction and a posterior-anterior direction relative to the longitudinal direction of the device. For example, inferior bone fastening borehole 30 may be angled at approximately 20 degrees in the inferior-superior direction and approximately 20 degrees in the posterior-anterior direction. As a result, when device 10 is secured to the medial cuneiform 6a, the inferior bone fastening borehole 30 is adapted to guide a bone screw through the medial cuneiform 6a and into the second metatarsal 4b. Each of the bone fastening boreholes 30 may have a diameter of approximately 7 mm. In a preferred embodiment, the bone fastening boreholes 30 may also include one or more cutting slots 36 sized to receive a cutting tool such as a scalpel.

Adjustment borehole 32 is preferably positioned at the midline of the device and relatively closer to anterior corner 26 than posterior surface 22. Adjustment borehole 32 is sized and shaped to receive a distal tip of an adjustment device such as forceps. In an exemplary embodiment, adjustment borehole 32 is approximately 3.3 mm in diameter.

Alignment index 34 is any mechanism for referencing proper alignment of device 10 with respect to a foot bone of a patient. Alignment index 34 may include, without limitation, indicia on a surface of the device, such as a reference line or an arrow, one or more markers for navigation, a side hole extending through lateral surface 16, two points provided at appropriate locations to establish a line or axis, or any combination of the foregoing to position and align the device on the foot of the patient. It will be appreciated that the indicia, the markers and/or the points may include a radiopaque marker at the appropriate location(s), and for example, may mirror the position of the below described side hole.

In robotic situations, navigation may also be used and alignment may be made via a pre-operative planning step useable with any of the alignment indexes but particularly with a robotic placement.

In situations in which alignment index 34 is a side hole, the side hole may extend through device 10 in a transverse direction (e.g., not parallel) to the longitudinal axis of the device. For example, side hole 34 may extend in an anterior-posterior direction from anterior corner 26 to adjustment borehole 32. Side hole 34 may be between approximately 1.6-2.0 mm in diameter and configured to receive a guidewire such as a k-wire. It will be appreciated that the locations of the at least one pin fixation borehole 28, the at least one bone fastening borehole 30, the adjustment tool borehole 32 and the side hole 34, as well as the sizes and angles of the same, may be modified for treating fractures and/or dislocations elsewhere on the foot or body.

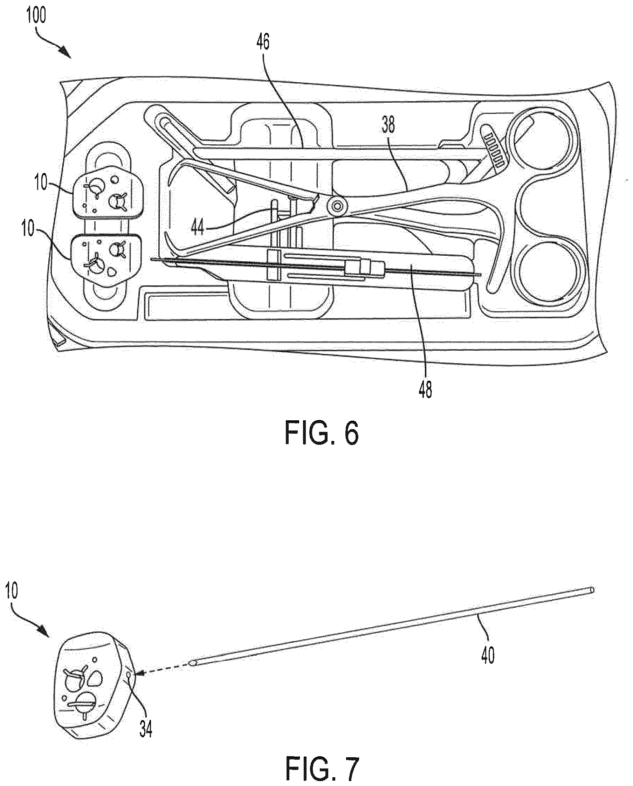

FIG. 6 illustrates a kit 100 for performing a Lisfranc surgery. The kit may be provided as a sterilized and disposable kit that includes all of the necessary components for performing a Lisfranc surgery. For example, kit 100 may include one or more alignment and fixation devices 10. Preferably, two devices 10 are included in each kit 100, a left device for treating a Lisfranc injury to the left foot and a mirror image right device for treating a Lisfranc injury to a right foot. Kit 100 may also include an adjustment tool such as forceps 38, a plurality of guidewires 40 (shown in FIG. 7), a pin 42 (shown in FIG. 9) for pinning the device to the foot of a patient, one or more bone fasteners such as bone screws 44, a wire guide 46, a depth gauge 48 and a scalpel (not shown).

Use of the kit 100 in treating a Lisfranc injury will now be described with reference to FIGS. 7-13. In prepping for a Lisfranc surgery, a surgeon or other user may retrieve a desired one of the right or left devices and a first guidewire 40 from kit 100. As shown in FIG. 7, first guidewire 40 is inserted through side hole 34 until the distal end of the guidewire is positioned within adjustment borehole 32.

The surgeon may then place a second guidewire 40 percutaneously into the cuneonavicular joint of the patient's foot to establish a posterior limit for the alignment and fixation device and a third guidewire percutaneously adjacent an inferior end of the medial cuneiform to establish an inferior limit of the alignment and fixation device. As shown in FIG. 8, the surgeon than places the concave bone contacting surface 12 of device 10 against the patient's skin, adjacent the medial cuneiform 6a, with the posterior surface 22 of the device against the second guidewire and the inferior surface 20 of the device against the third guidewire. The surgeon may then adjust the device until the first guidewire is aligned parallel to the longitudinal axis of the first metatarsal 4a. It will be appreciated that in other embodiments including other alignment indexes 34 (not shown), device 10 may be properly positioned by aligning the alignment index in parallel to the longitudinal axis of the first metatarsal 4a or at another desired position relative to another bone. For example, if the indicia is a reference line provided on a surface of the device and extending in the direction of the side hole, the surgeon may align the reference line along the longitudinal axis of the first metatarsal. Alternatively, if the indicia is an array of markers, the surgeon may manually align the markers or use known navigation techniques in concert with the assistance of a surgical robot to align the markers and the midline of the device along the first metatarsal of the patient.

One or more pins 42 are then inserted through pin fixation boreholes 28, through the patient's skin and into bone to temporarily secure the device to the mid-foot of the patient. The pins may be bent or trimmed, as shown in FIG. 9, in order to improve access to bone fastening boreholes 30 and adjustment borehole 32.

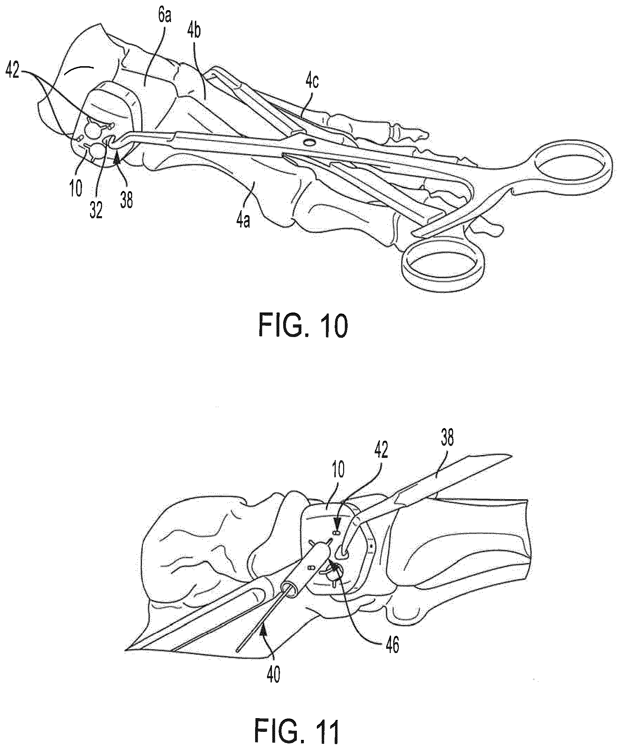

After the device has been secured to the foot of the patient, the surgeon may then retrieve forceps 38 from kit 100. As shown in FIG. 10, a first tip of the forceps is inserted through adjustment borehole 32 and placed into engagement with the medial cuneiform 6a. The second tip of the forceps is placed between the second and third metatarsals and into engagement with the second metatarsal. The forceps may then be clamped until satisfactory reduction of the Lisfranc complex has been achieved.

As shown in FIG. 11, a wire guide device 46 is then retrieved from kit 100 and inserted into one of the bone fastening boreholes 30, for example, the superior bone fastening borehole. A guidewire 40 is then inserted through wire guide device 46 and into the skin of the patient. After guidewire 40 has been placed in position, wire guide device 46 is removed and a cannulated drill is placed over the guidewire and used to drill a pilot hole into the medial cuneiform 6*a*, intermediate cuneiform 6*b* and lateral cuneiform 6*c*. Depth gauge 48 may then be retrieved from kit 100 and used to verify that the pilot hole has been drilled to a desired depth. The guidewire 40 may then be removed and a cutting device, such as a scalpel, may be inserted through the cutting slots 36 of superior bone fastening borehole 30 to make small incisions in the patient's skin in order to accommodate seating of the head of bone screw 44. It will be appreciated that the patient's skin may be incised at any time after device 10 has been secured to the foot of the patient and before the bone screw has been inserted into bone, for example, before or after the drilling of the pilot hole. With the desired bone screw in hand, the surgeon may then insert the bone screw entirely through the superior bone fastening borehole 30 and into position to secure the medial cuneiform 6*a*, intermediate cuneiform 6*b* and lateral cuneiform 6*c*. If bone screw 44 is a cannulated screw, the cannulated bone screw may instead be inserted over the guidewire 40 before the guidewire is removed.

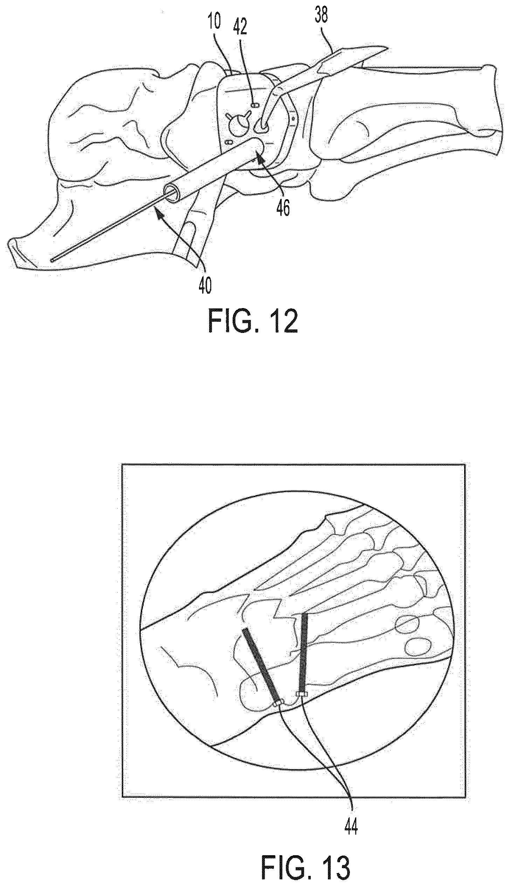

The surgeon may then insert guidewire device 46 into the inferior bone fastening borehole, as shown in FIG. 12, and repeat the process described above. More specifically, guidewire 40 may be inserted through guidewire device 46 to a desired depth. Guidewire device 46 may then be removed and the cannulated drill may be used to drill a pilot hole through the medial cuneiform 6*a* and into the second metatarsal. Depth gauge 48 may again be used to verify that the pilot hole was drilled to a desired depth and to assist the user in choosing a bone screw 44 of proper length. A scalpel, or other cutting device, may be inserted through the cutting slots 36 of inferior bone fastening borehole 30 to make small incisions in the patient's skin, either before or after the pilot hole has been drilled. The surgeon may then insert another bone screw entirely through the device and into bone to secure the medial cuneiform 6*a* and second metatarsal 4*b*.

FIG. 13 is an x-ray illustrating the positions of bone screws 44 within the patient's treated foot. It will be appreciated that bone screws 44 may be inserted into their respective bones in either order. That is, the bone screw may be inserted and guided through the superior bone fastening borehole either before or after the bone screw is inserted and guided through the inferior bone fastening borehole. After bone screws 44 have been fastened, pins 42 are then removed and device 10 is detached from the patient's foot. One or more bone plates (not shown) may then optionally be secured to the foot of the patient, if necessary, to provide extra support to the treatment area.

The above described embodiment is but one exemplary embodiment specially adapted for guiding an inter-cuneiform bone screw 44 into the medial cuneiform 6*a*, intermediate cuneiform 6*b* and lateral cuneiform 6*c*, and for guiding a Home Run screw through the medial cuneiform 6a and the second metatarsals 4*b*. Nevertheless, the position and angles of the pin fixation boreholes 28, bone fastening boreholes 30, adjustment borehole 32 and side borehole 34 may be modified depending upon the device's application.

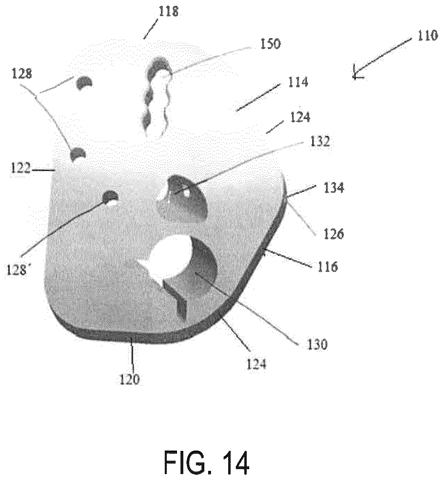

An exemplary modification, as shown in FIG. 14, illustrates an alignment and fixation device 110 in accordance with another embodiment for realigning and fixating displaced bone during a Lapidus procedure. Alignment and fixation device 110 is substantially similar to alignment and fixation device 10, and thus, specific features of device 110 may not described again hereinafter in detail, unless the features are emphasized or unless the features are different than the features previously described with respect to device 10. Instead, when like features are mentioned, the features are renumbered with sequential 100 series numerals. Device 110 has a bone contacting surface 112 (which may be concave), an opposing surface (not shown and "which may be convex), a lateral surface 116 disposed between the bone contacting surface and the opposing surface and a longitudinal axis passing through the bone contacting surface and the opposing surface. Device 110 may be pentagonal in shape such that lateral surface 116 defines a superior surface 118, an inferior surface 120, a posterior surface 122, angled surfaces 124 and an anterior corner 126 for assisting a user in positioning device 110 in the proper location on the patient's midfoot.

Like device 10, device 110 defines at least one pin fixation borehole 128, at least one bone fastening borehole 130, an adjustment tool borehole 132 and an alignment index 134 for properly positioning the body of the device on the foot of the patient. Device 110 includes at least two pin fixation boreholes 128. While pin fixation boreholes 128 may be positioned at any location on device 110, an alignment pin fixation borehole 128' is preferably positioned at the midline of the device and adjacent the posterior surface 122 of the body for assisting in the alignment of the device as discussed below.

Bone fastening borehole 130, adjustment tool borehole 132 and alignment index 134 of device 110 are generally similar to bone fastening borehole 30, adjustment tool borehole 32 and alignment index 34 of device 10, and therefore, are not discussed again in detail. It is noted, however, that bone fastening borehole 130 is more akin to the inferior bone fastening borehole of device 10 than the superior bone fastening borehole. It will also be appreciated that while alignment index 134 is again illustrated as a side hole, the alignment index may also be any of the alternative alignment indexes discussed above with respect to alignment and fixation device 10.

Alignment and fixation device 110 further include a rotation slot 150 extending completely through the body of the device from the opposing surface 114 of the device to the bone contacting surface of the device. Rotation slot 150 is preferably positioned approximately halfway between the posterior surface 122 and the anterior corner 126 of device 110 and extends from a location adjacent superior surface 118 toward inferior surface 120 of the device. However, rotation slot 150 need not be positioned approximately halfway between posterior surface 122 and anterior corner 126 of device 110 so long as the rotation slot is at least partially positioned anterior the alignment pin fixation borehole 128'. In one exemplary embodiment, rotation slot 150 is formed by a series of interconnecting holes extending in the superior-inferior direction. Each one of the holes being sized and configured to sequentially receive pin, or similar tool, as described in further detail below.

Use of alignment and fixation device 110 in performing a Lapidus bunionectomy will now be described. Initially, a first guidewire 140 is inserted through side hole 134 until the distal end of the guidewire is positioned within adjustment borehole 132. The surgeon may then place a second guidewire 140 percutaneously into the first tarsometatarsal joint of the patient's foot before sliding device 110 over the second guidewire (such that the second guidewire is slid through alignment pin fixation borehole 128' and the bone contacting surface 112 of device 110 contacts the skin of the patient). Device 110 may then be rotated, as necessary, to align the first guidewire with the bisection of the first metatarsal 4a. Of course, device 110 may be alternatively aligned using any of the aforementioned alignment indexes 134.

After device 110 has been properly positioned and aligned on the foot of the patient, the surgeon may insert fixation pins 142 into pin fixation boreholes 128, through the patient's skin and into bone to temporarily secure the device to the mid-foot of the patient. The pins may be bent or trimmed in order to improve access to bone fastening borehole 130, adjustment borehole 132 and rotation slot 150.

A first tip of the forceps may then be inserted through adjustment borehole 132 and placed into engagement with bone while a second tip of the forceps is placed between the second and third metatarsals and into engagement with the second metatarsal. The forceps are then clamped until sufficient reduction has been achieved (e.g., lateral displacement of the first metatarsal 4a toward the second metatarsal).

A pin, or pin-like device, may then be inserted through one of the holes forming rotation slot 150 at a location superior to the first metatarsal 4a. The pin may then be used in a similar manner as a joystick and pulled in an inferior direction for rotating the first metatarsal and correcting the frontal plane alignment of the sesamoid complex. If further adjustment is necessary, the pin may then be moved to another one of the holes that forms rotation slot 150, and that is located inferiorly to the hole in which the previous adjustment was made, before the pin is again pulled in the inferior direction. This process may be repeated as many times as necessary until sufficient frontal plane correction has been achieved. It will be appreciated that the frontal plane correction may be performed before, concurrently or after the reduction step.

After first metatarsal 4a has been properly realigned relative to medial cuneiform 6a, bone screw may be inserted through bone fastening borehole (e.g., with the assistance of guidewire, wire guide, depth gauge, scalpel and a cannulated drill) as described above with respect to device 10. Specifically, bone screw may be inserted through the first metatarsal and through cuneiform bone to fixate the realigned bone in place.

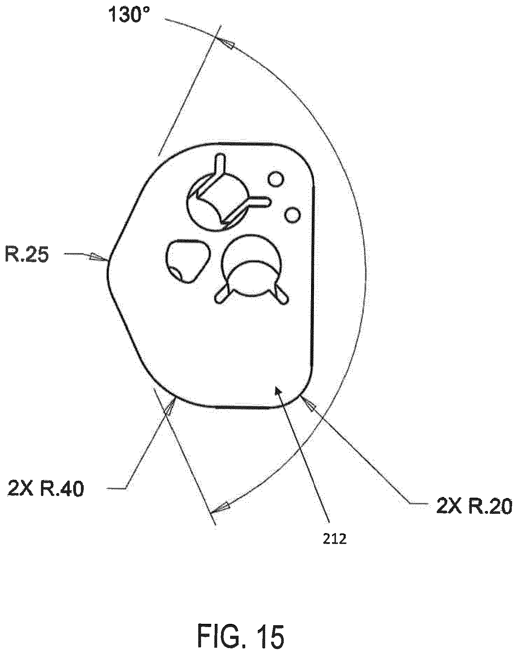

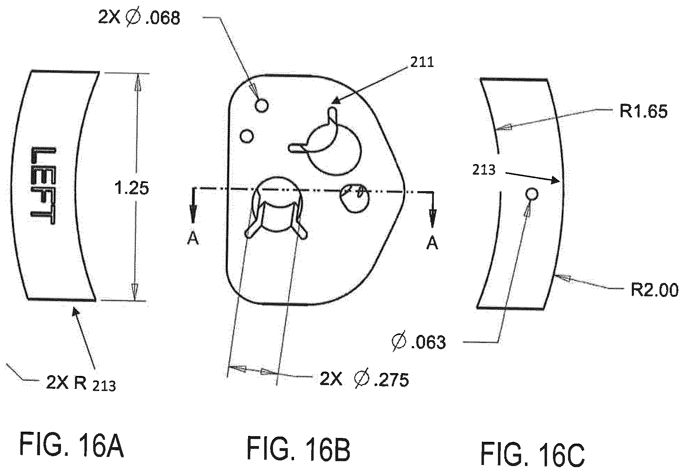

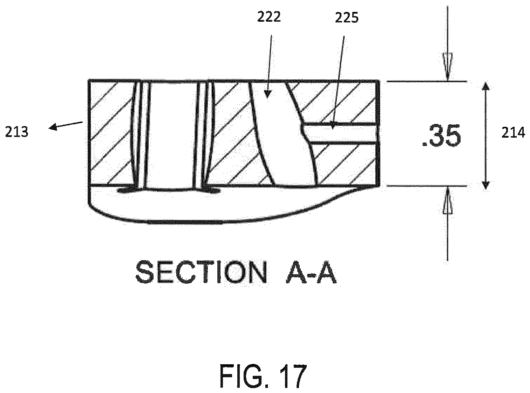

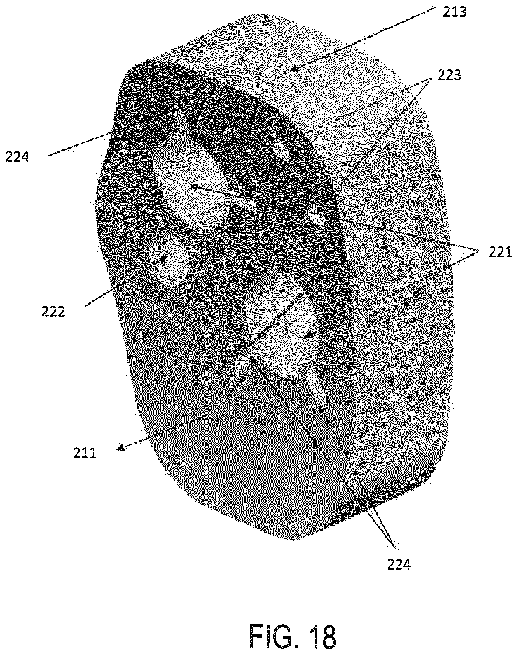

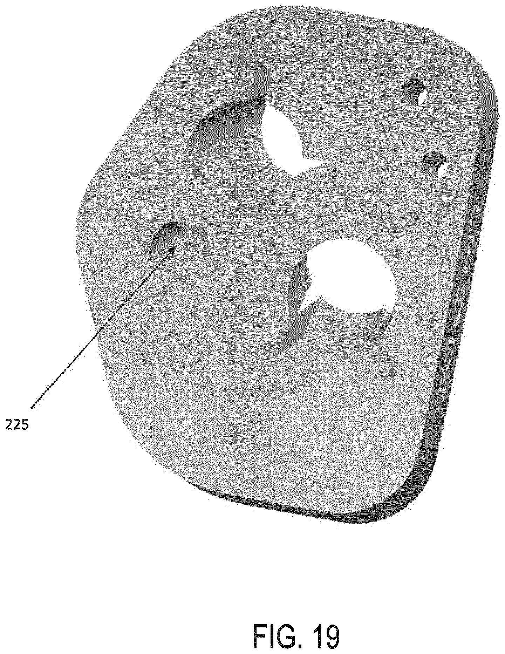

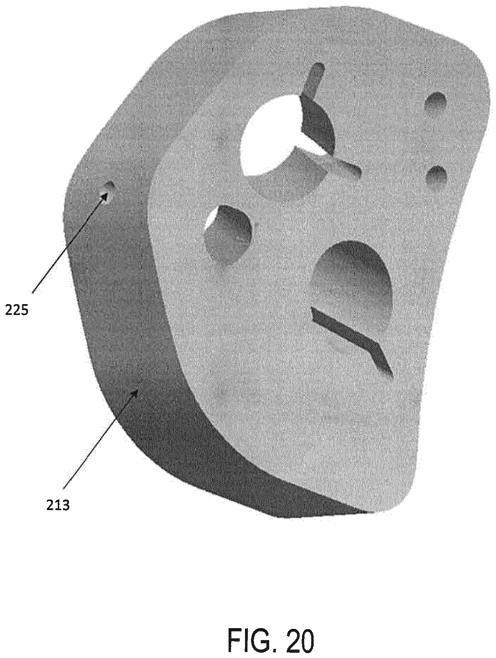

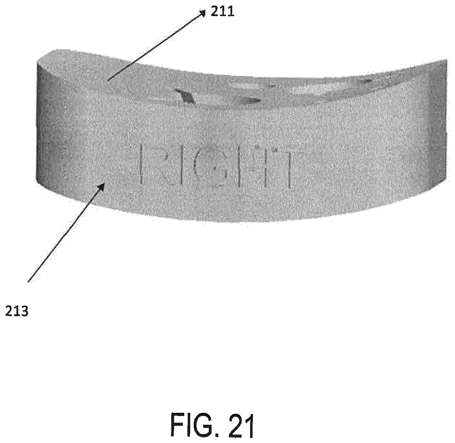

With reference to Figures 15-21, a further embodiment of the disclosed fixation device 201 comprises a top surface 211 and a bottom surface 212. The curvatures of the top and bottom surfaces 211, 212 are selected in accordance with the anatomical area to be treated. For a fixation device 201 used during open reduction of a Lisfranc injury, the top surface 211 is preferably a concave surface, and the bottom surface 212 is preferably a convex surface, or *vice versa.*

In an embodiment, the top surface 211 has a first radius of curvature R1, and the bottom surface 212 has a second radius of curvature R2. R1 can be the same or different from R2. In an embodiment, RI is smaller than R2. In another embodiment, R2 is smaller than RI. In yet another embodiment, the top 211 and/or the bottom surface 212 is substantially flat.

The top surface 211 and the bottom surface 212 are connected by a lateral surface 213, the height of which represents a thickness 214 of the Chip 201, which thickness 214 can be constant or variable. That is, thickness 214 of the Chip 201 from the top surface 211 to the bottom surface 212 can be constant or variable around the entirety of the Chip 201.

The Chip 201 further comprises at least 5 main through holes extending through from the top surface 211 to the bottom surface 212, thus forming a tunnel having a first opening in the top surface 211 and a second opening in the bottom surface 212.

The 5 preferably substantially vertical main through holes preferably include holes of at least three different shapes and/or sizes. In an embodiment, the main through holes including two of a first size and/or shape ("the first type" designated 221) for guiding holes for Home Run (medial cuneiform to second metatarsal base screw) and intercuneiform screws, one of a second size and/or shape ("the second type" designated 222) for receiving tips of bone reduction forceps, and two of a third size and/or shape ("the third type" designated 223) for receiving temporary fixation pins, and. In an embodiment, holes of the first type 221 have diameters larger than that of the second type 222. In another embodiment, holes of the second type 222 of diameters larger than that of the third type 223. In another embodiment, holes of the first type 221 and the second type 222 have parallel longitudinal axis. In another embodiment, holes of the second type 222 and the third type 223 have parallel longitudinal axis. In another embodiment, holes of the first type 221 and the third type 223 have parallel longitudinal axis. In an embodiment, said longitudinal axis is substantially parallel to the thickness 214 direction of the Chip 201.

In a further embodiment, the Chip 201 further comprises one or more, for example two, slots 224 on and/or around the openings of holes of the first type 221, which slots 224 provide clearance for scalpel cuts prior to screw insertion. Similar to the main through holes, the slots can run through from the top surface 211 to the bottom surface 212.

The Chip 201 also comprises a preferably substantially horizontal side through hole 225 extending from the lateral surface 213 through to one of the main through holes, thus forming a tunnel having a first opening on the lateral surface 213 and second opening in a wall of a main through hole tunnel. Preferably, the side through hole 225 forms a tunnel connecting the lateral surface 213 of the Chip 201 to a wall of the tunnel of the hole of the second type 222. In one embodiment, the side through hole 225 is adapted to receive guide wire for anatomical alignment with 1st ray of foot.

In an embodiment, the Chip 201 is in the shape a pentagonal prism, wherein the top and bottom surfaces 211, 212 are substantially in the shape of a pentagon having preferably rounded corners (i.e., no sharp, pointed vertices). Preferably, the shapes of the top and bottom surfaces 211, 212 are convex polygons, e.g., pentagons. The top and bottom surfaces 211, 212 can be independently in a shape of a regular polygons or irregular polygons. Further, the shapes of the top and bottom surfaces 211, 212 can be independently symmetrical or asymmetrical. The shapes of the top and bottom surfaces 211, 212 are selected in accordance with the anatomical area to be treated.

The Chip 201 can be made from various different materials, preferably polymeric or thermoplastic materials. Suitable materials include but is not limited to glass-fiber reinforced grade of polyacrylamide (PARA) such as Ixef^{®} GS-1022 Polyarylamide (PARA) available from Solvay Specialty Polymers USA, L.L.C. (Alpharetta, GA USA).

In a particular embodiment, a fixation device 201 for orthopedic surgery is provided, comprising a) a top surface 211, b) a bottom surface 212, c) a lateral surface 213 connecting the top surface 211 and the bottom surface, d) at least five, preferably five main through holes extending from the top surface 211 to the bottom surface 212, so as to form five main through hole tunnels each having a first opening in the top surface 211 and a second opening in the bottom surface 212, and e) a side through hole extending from the lateral surface 213 to a wall of a tunnel formed by one of the main through holes, so as to form a side through hole 225 tunnel having a first opening in lateral surface 213 and a second opening in the wall of a main through hole tunnel.

In one embodiment, the side through hole has a longitudinal axis substantially perpendicular to that of one or more of the main through holes. In another embodiment, at least one of the main through holes has a longitudinal axis substantially parallel to that of one or more of the other main through holes. In another embodiment, each of the main through holes has a longitudinal axis substantially parallel to that of one or more of the other main through holes. In another embodiment, each of the main through holes has a longitudinal axis substantially parallel to that of each of the other main through holes. In another embodiment, the main holes are of at least 3 different shapes and/or different sizes. In another embodiment, the top surface is a concave surface. In another embodiment, the bottom surface is a convex surface. In another embodiment, the top surface has a first radius of curvature RI, and the bottom surface has a second radius of curvature R2, and RI is the same or different from R2. In another embodiment, RI is smaller than R2. In another embodiment, R2 is smaller than RI. In another embodiment, the top and/or the bottom surface has a symmetric shape. In another embodiment, the top and/or the bottom surface has an asymmetric shape. In another embodiment, the top and/or the bottom surface have the same shape and/or are the same size. In another embodiment, the top and/or the bottom surface have different shapes and/or different sizes. In yet another embodiment, the fixation device is in the shape of a polygonal prism, preferably a pentagonal prism.

A kit comprising one, two, or more of the fixation devices 201 as describe hereinabove is also provided. Preferably, wherein two Chips 201 are included in the kit, they are of mirror images of each other (i.e., a "left" Chip and a "right" Chip). The kit is preferably provided as a sterile and/or disposable kit.

It is contemplated that the Chip 201 described herein can be provided in a range of sizes and shapes depending on the body part being treated.

In an embodiment, the Chip 201 can be about 15-200 or 15-100 mm in length. In another embodiment, the Chip 201 can be about 15-200 mm in width. In another embodiment, the Chip 201 can be about 32 mm long. In another embodiment, the Chip 201 can be about 25 mm wide.

In another embodiment, the Chip 201 can be about 5-100 mm thick. In another embodiment, the Chip 201 can be about 9 mm thick. In another embodiment, the Chip 201 can be about 12 mm thick.

In another embodiment, the first type of holes 221 has a cross-sectional diameter of about 7 mm. In another embodiment, the second type of holes 222 has a cross-sectional diameter of about 3.3 mm. In yet another embodiment, the third type of holes 223 has a cross-sectional diameter of 1.6 mm. The cross-sectional diameters of the various types of holes are selected in accordance with the anatomical area to be treated.

In another embodiment, the Chip 201 as described herein and/or a kit comprising the same can be used during surgical treatment of an orthopedic injury as follows:
1) The Chip 201 is temporarily pinned to the skin of the patient;
2) Forceps (fracture reduction or bone re-alignment) are attached to the corresponding bone andalso attached to the Chip 201 through the appropriate hole;
3) Guide sheath is placed in the appropriate holes of the Chip 201. Pins or drill bits are placed into the guide sheath and the fractures or bone re-alignment cuts are created using the Chip 201 as a guide;
4) Screws are placed through the Chip 201 and the repair is maintained; and
5) The Chip 201 is removed from the skin and disposed of.

Finally, it should be clear to those skilled in the art that each embodiment disclosed herein can be and is contemplated as being applicable to each of the other disclosed embodiments. In particular, the elements described herein with respect to the fixation device 201 can be applicable in the method of use and/or kit embodiments described herein and *vice versa.* Accordingly, all combinations of the various elements described herein are within the scope of the invention.

### Advantages of the fixation device

The fixation device 201 as described above is adapted for use during surgical treatment of orthopedic injury or fracture. Specifically, the Chip 201 is placed on the portion of the body where the injury has occurred. It is held in place with temporary pinning and the holes in the Chip 201 are strategically located depending on the specific orthopedic injury and fracture to be treated. The Chip 201 can be used in all fractures and dislocations of the lower and upper extremities. Additionally, the Chip 201 includes areas where fracture reduction tools can be applied so that injuries can be repaired anatomically. Multiple screws and pins can be placed into the body using the Chip 201 as a guide allowing for direct and anatomic repair of injury to the upper or lower extremities. The Chip 201 does not need to be removed from the skin during the surgical repair to assure accurate placement of hardware. The Chip 201 differs from other available systems in that it is single use and disposable. This prevents wear and tear to the reduction device during the process of sterilization, which prevents the device from becoming misshaped; which again improves accuracy during the surgical procedure.

The Chip 201 can be used when treating various injuries and/or fractures, including talar fractures, navicular fractures, cuneiform fractures, cuboid fractures, metatarsal base fractures, proximal and distal tibial fractures, distal radius fracture, fractures of the carpal bones, midfoot arthroplasty, hindfoot arthroplasty, and osteotomies of the hindfoot, midfoot and ankle. The Chip 201 can also be used to treat tibial plateau fractures and create proximal and distal tibial osteotomies.

To summarize the foregoing, a fixation device for orthopedic surgery, comprising a) a top surface, b) a bottom surface, c) a lateral surface connecting the top surface and the bottom surface, d) at least five main through holes extending from the top surface to the bottom surface, so as to form at least five main through hole tunnels each having a first opening in the top surface and a second opening in the bottom surface, and e) a side through hole extending from the lateral surface to a wall of a tunnel formed by one of the main through holes, so as to form a side through hole tunnel having a first opening in lateral surface and a second opening in the wall of a main through hole tunnel; and/or the side through hole has a longitudinal axis substantially perpendicular to that of one or more of the main through holes; and/or further comprising at least one of the main through holes has a longitudinal axis substantially parallel to that of one or more of the other main through holes; and/or each of the main through holes has a longitudinal axis substantially parallel to that of one or more of the other main through holes; and/or each of the main through holes has a longitudinal axis substantially parallel to that of each of the other main through holes; and/or the main holes are of at least 3 different shapes and/or different sizes; and/or the top surface is a concave surface; and/or the bottom surface is a convex surface; and/or the top surface has a first radius of curvature R1, and the bottom surface has a second radius of curvature R2, and R1 is the same or different from R2; and/or R1 is smaller R2 is smaller than R1; and/or the top and/or the bottom surface has a symmetric shape; and/or the top and/or the bottom surface has an asymmetric shape; and/or the top and/or the bottom surface have the same shape and/or are the same size; and/or the top and/or the bottom surface have different shapes and/or different sizes; and/or in the shape of a polygonal prism, preferably a pentagonal prism,

A kit comprising the fixation device; and/or comprising at least two of the fixation device.

A method of performing surgery to treat an orthopedic injury and/or fracture in a patient, comprising a step of temporarily applying the fixation device to the site of the orthopedic injury and/or fracture on the skin of the patient; and/or comprising the steps of a) pinning the fixation device to the skin of the patient, b) attaching a forceps to the fixation device through a hole therein, and attaching said forceps to a bone of the patient, c) placing a guide sheath in a hole of the fixation device, d) placing pins or drill bits into the guide sheath, e) making cuts using the fixation device as guide, f) placing screws through the fixation device, and g) remove the fixation device from the skin of the patient.

Although the invention herein has been described with reference to particular embodiments, it is to be understood that these embodiments are merely illustrative of the principles and applications of the present invention. It is therefore to be understood that numerous modifications may be made, and are encouraged to be made, to the illustrative embodiments and that other arrangements may be devised without departing from the present invention as defined by the appended claims.

## Claims

1. An alignment and bone fixation device (10, 110) for use in orthopedic surgery of a foot and/or ankle bone, comprising:
a body having a bone facing surface (12, 112), an opposing surface (14, 114), a lateral surface (16, 116) disposed between the bone facing surface and the opposing surface, and a longitudinal axis extending through the bone facing surface and the opposing surface, the body having a first pin hole (28, 128) extending through the body from the bone facing surface to the opposing surface and being sized and configured to receive a pin (42, 142), **characterised by** comprising:
a tool hole (32, 132) extending at least partially through the body and configured to receive an adjustment tool (38) for aligning bone, a first bone fastener hole (30, 130) extending through the body from the bone facing surface to the opposing surface sized and configured to guide a bone fastener (44) entirely through the device to secure aligned bone, and an alignment index (34, 134) establishing an axis transverse to the longitudinal axis of the body for positioning the body on the foot and/or ankle bone by aligning the axis of the alignment index and a longitudinal axis of the bone.

2. The alignment and bone fixation device (10, 110) of claim 1, wherein the bone facing surface (12, 112) is concave relative to a plane passing between the bone facing surface and the opposing surface (14, 114) in a direction orthogonal to the longitudinal axis.

3. The alignment and bone fixation device (10, 110) of claim 1 or 2, wherein the opposing surface (14, 114) is convex relative to a plane passing between the bone facing surface (12, 112) and the opposing surface in a direction orthogonal to the longitudinal axis.

4. The alignment and bone fixation device (10, 110) of any of the preceding claims, wherein the body further defines a second pin hole (28, 128) extending through the body from the bone facing surface (12, 112) to the opposing surface (14, 114).

5. The alignment and bone fixation device (10, 110) of any of the preceding claims, wherein the body further defines a second bone fastener hole (30, 130) extending through the body from the bone facing surface (12, 112) to the opposing surface (14, 114).

6. The alignment and bone fixation device (10, 110) of any of the preceding claims, wherein the first bone fastener hole (30, 130) comprises at least one cutting slot (36) adapted to receive a cutting tool.

7. The alignment and bone fixation device (10, 110) of any of the preceding claims, wherein the body is substantially pentagonal in shape and the lateral surface (16, 116) defines a superior surface (18, 118), an inferior surface (20, 120), a posterior surface (22, 122) and an anterior corner (26, 126).

8. The alignment and bone fixation device (10, 110) of claim 7, wherein the alignment index (34, 134) is at least one of indicia, a radiopaque marker or markers, a navigation marker or markers, and/or a side hole.

9. The alignment and bone fixation device (10, 110) of claim 8, wherein the side hole is disposed at the anterior corner (26, 126) and extends in a direction substantially orthogonal to the longitudinal axis.

10. The alignment and bone fixation device (10, 110) of claim 8 or 9, wherein the side hole intersects the tool hole (32, 132).

11. The alignment and bone fixation device (10, 110) of any of the preceding claims, wherein the tool hole (32, 132) extends completely through the body from the bone facing surface (12, 112) to the opposing surface (14, 114).

12. The alignment and bone fixation device (10, 110) of any of the preceding claims, further comprising a rotation slot (150) formed from a plurality of interconnecting holes extending in the superior-inferior direction.

13. A kit (100) for orthopedic surgery of a foot and/or ankle bone, comprising:
at least one alignment and bone fixation device (10, 110) of any of the preceding claims; and
forceps (38) for aligning displaced bone.

14. The kit (100) of claim 13, further comprising at least one of a guidewire (40), a pin (42), a bone screw (44), a wire guide (46), a depth gauge (48) and/or a scalpel.

15. The kit (100) of claim 13 or 14, wherein the at least one alignment and bone fixation device (10, 110) includes a first alignment and bone fixation device and a second alignment and bone fixation device, the first and second alignment and bone fixation devices being mirror images of one another.

## Patentansprüche

1. Ausrichtungs- und Knochenfixierungsvorrichtung (10, 110) zur Verwendung in der orthopädischen Chirurgie eines Fuß- und/oder Knöchelknochens, umfassend:
einen Körper mit einer dem Knochen zugewandten Oberfläche (12, 112), einer gegenüberliegenden Oberfläche (14, 114), einer seitlichen Oberfläche (16, 116), die zwischen der dem Knochen zugewandten Oberfläche und der gegenüberliegenden Oberfläche angeordnet ist, und einer Längsachse, die sich durch die dem Knochen zugewandte Oberfläche und die gegenüberliegende Oberfläche erstreckt, wobei der Körper ein erstes Stiftloch (28, 128) aufweist, das sich durch den Körper von der dem Knochen zugewandten Oberfläche zu der gegenüberliegenden Oberfläche erstreckt und so bemessen und konfiguriert ist, dass es einen Stift (42, 142) aufnehmen kann,
**dadurch gekennzeichnet, dass** sie umfasst:
ein Werkzeugloch (32, 132), das sich zumindest teilweise durch den Körper erstreckt und zur Aufnahme eines Ausrichtwerkzeugs (38) zum Ausrichten von Knochen konfiguriert ist, ein erstes Knochenbefestigungsloch (30, 130), das sich durch den Körper von der dem Knochen zugewandten Oberfläche zu der gegenüberliegenden Oberfläche erstreckt und so bemessen und konfiguriert ist, dass es ein Knochenbefestigungselement (44) vollständig durch die Vorrichtung führt, um den ausgerichteten Knochen zu sichern, und einen Ausrichtungsindex (34, 134), der eine Achse quer zu der Längsachse des Körpers festlegt, um den Körper am Fuß- und/oder Knöchelknochen zu positionieren, indem die Achse des Ausrichtungsindex und eine Längsachse des Knochens ausgerichtet werden.

2. Ausrichtungs- und Knochenfixierungsvorrichtung (10, 110) nach Anspruch 1, wobei die dem Knochen zugewandte Oberfläche (12, 112) relativ zu einer Ebene, die zwischen der dem Knochen zugewandten Oberfläche und der gegenüberliegenden Oberfläche (14, 114) in einer Richtung orthogonal zu der Längsachse verläuft, konkav ist.

3. Ausrichtungs- und Knochenfixierungsvorrichtung (10, 110) nach Anspruch 1 oder 2, wobei die gegenüberliegende Oberfläche (14, 114) relativ zu einer Ebene, die zwischen der dem Knochen zugewandten Oberfläche (12, 112) und der gegenüberliegenden Oberfläche in einer Richtung orthogonal zu der Längsachse verläuft, konvex ist.

4. Ausrichtungs- und Knochenfixierungsvorrichtung (10, 110) nach einem der vorhergehenden Ansprüche, wobei der Körper ferner ein zweites Stiftloch (28, 128) definiert, das sich durch den Körper von der dem Knochen zugewandten Oberfläche (12, 112) zu der gegenüberliegenden Oberfläche (14, 114) erstreckt.

5. Ausrichtungs- und Knochenfixierungsvorrichtung (10, 110) nach einem der vorhergehenden Ansprüche, wobei der Körper ferner ein zweites Knochenbefestigungsloch (30, 130) definiert, das sich durch den Körper von der dem Knochen zugewandten Oberfläche (12, 112) zu der gegenüberliegenden Oberfläche (14, 114) erstreckt.

6. Ausrichtungs- und Knochenfixierungsvorrichtung (10, 110) nach einem der vorhergehenden Ansprüche, wobei das erste Knochenbefestigungsloch (30, 130) mindestens einen Schneideschlitz (36) aufweist, der zur Aufnahme eines Schneidewerkzeugs geeignet ist.

7. Ausrichtungs- und Knochenfixierungsvorrichtung (10, 110) nach einem der vorhergehenden Ansprüche, wobei der Körper im Wesentlichen fünfeckig ist und die seitliche Oberfläche (16, 116) eine superior gelegene Oberfläche (18, 118), eine inferior gelegene Oberfläche (20, 120), eine posterior gelegene Oberfläche (22, 122) und eine anterior gelegene Ecke (26, 126) definiert.

8. Ausrichtungs- und Knochenfixierungsvorrichtung (10, 110) nach Anspruch 7, wobei der Ausrichtungsindex (34, 134) mindestens eines der folgenden Elemente ist: Indizien, eine röntgendichte Markierung oder Markierungen, eine Navigationsmarkierung oder Markierungen und/oder ein Seitenloch.

9. Ausrichtungs- und Knochenfixierungsvorrichtung (10, 110) nach Anspruch 8, wobei das Seitenloch an der anterior gelegenen Ecke (26, 126) angeordnet ist und sich in einer Richtung im Wesentlichen orthogonal zu der Längsachse erstreckt.

10. Ausrichtungs- und Knochenfixierungsvorrichtung (10, 110) nach Anspruch 8 oder 9, wobei das Seitenloch das Werkzeugloch (32, 132) schneidet.

11. Ausrichtungs- und Knochenfixierungsvorrichtung (10, 110) nach einem der vorhergehenden Ansprüche, wobei sich das Werkzeugloch (32, 132) vollständig durch den Körper von der dem Knochen zugewandten Oberfläche (12, 112) zu der gegenüberliegenden Oberfläche (14, 114) erstreckt.

12. Ausrichtungs- und Knochenfixierungsvorrichtung (10, 110) nach einem der vorhergehenden Ansprüche, ferner umfassend einen Rotationsschlitz (150), der aus einer Vielzahl von miteinander verbundenen Löchern gebildet ist, die sich in der superior-inferior Richtung erstrecken.

13. Kit (100) für die orthopädische Chirurgie eines Fuß- und/oder Knöchelknochens, umfassend:
mindestens eine Ausrichtungs- und Knochenfixierungsvorrichtung (10, 110) nach einem der vorhergehenden Ansprüche; und
eine Zange (38) zum Ausrichten verschobener Knochen.

14. Kit (100) nach Anspruch 13, ferner umfassend mindestens einen Führungsdraht (40), einen Stift (42), eine Knochenschraube (44), eine Drahtführung (46), einen Tiefenmesser (48) und/oder ein Skalpell.

15. Kit (100) nach Anspruch 13 oder 14, wobei die mindestens eine Ausrichtungs- und Knochenfixierungsvorrichtung (10, 110) eine erste Ausrichtungs- und Knochenfixierungsvorrichtung und eine zweite Ausrichtungs- und Knochenfixierungsvorrichtung umfasst, wobei die erste und zweite Ausrichtungs- und Knochenfixierungsvorrichtung spiegelbildlich zueinander sind.

## Revendications

1. Dispositif (10, 110) d'alignement et de fixation d'os pour une utilisation en chirurgie orthopédique d'un os du pied et/ou de la cheville, comprenant :
un corps ayant une surface (12, 112) faisant face à l'os, une surface opposée (14, 114), une surface latérale (16, 116) disposée entre la surface faisant face à l'os et la surface opposée, et un axe longitudinal s'étendant à travers la surface faisant face à l'os et la surface opposée, le corps ayant un premier trou (28, 128) pour broche s'étendant à travers le corps depuis la surface faisant face à l'os jusqu'à la surface opposée et étant dimensionné et configuré pour recevoir une broche (42, 142), **caractérisé en ce que** comprenant :
un trou (32, 132) pour outil s'étendant au moins partiellement à travers le corps et configuré pour recevoir un outil (38) d'ajustement pour aligner un os, un premier trou (30, 130) pour dispositif de fixation d'os s'étendant à travers le corps depuis la surface faisant face à l'os jusqu'à la surface opposée dimensionné et configuré pour guider un dispositif (44) de fixation d'os entièrement à travers le dispositif pour immobiliser un os aligné, et un repère (34, 134) d'alignement établissant un axe transversal à l'axe longitudinal du corps pour positionner le corps sur l'os du pied et/ou de la cheville en alignant l'axe du repère d'alignement et un axe longitudinal de l'os.

2. Dispositif (10, 110) d'alignement et de fixation d'os selon la revendication 1, dans lequel la surface (12, 112) faisant face à l'os est concave par rapport à un plan passant entre la surface faisant face à l'os et la surface opposée (14, 114) dans un sens orthogonal à l'axe longitudinal.

3. Dispositif (10, 110) d'alignement et de fixation d'os selon la revendication 1 ou 2, dans lequel la surface opposée (14, 114) est convexe par rapport à un plan passant entre la surface (12, 112) faisant face à l'os et la surface opposée dans un sens orthogonal à l'axe longitudinal.

4. Dispositif (10, 110) d'alignement et de fixation d'os selon l'une quelconque des revendications précédentes, dans lequel le corps définit en outre un deuxième trou (28, 128) pour broche s'étendant à travers le corps depuis la surface (12, 112) faisant face à l'os jusqu'à la surface opposée (14, 114).

5. Dispositif (10, 110) d'alignement et de fixation d'os selon l'une quelconque des revendications précédentes, dans lequel le corps définit en outre un deuxième trou (30, 130) pour dispositif de fixation d'os s'étendant à travers le corps depuis la surface (12, 112) faisant face à l'os jusqu'à la surface opposée (14, 114).

6. Dispositif (10, 110) d'alignement et de fixation d'os selon l'une quelconque des revendications précédentes, dans lequel le premier trou (30, 130) pour dispositif de fixation d'os comprend au moins une fente (36) de coupe adaptée à recevoir un outil de coupe.

7. Dispositif (10, 110) d'alignement et de fixation d'os selon l'une quelconque des revendications précédentes, dans lequel le corps est de forme sensiblement pentagonale et la surface latérale (16, 116) définit une surface supérieure (18, 118), une surface inférieure (20, 120), une surface postérieure (22, 122) et un angle antérieur (26, 126).

8. Dispositif (10, 110) d'alignement et de fixation d'os selon la revendication 7, dans lequel le repère (34, 134) d'alignement est au moins un parmi des signes, un marqueur ou des marqueurs radio-opaques, un marqueur ou des marqueurs de navigation, et/ou un trou latéral.

9. Dispositif (10, 110) d'alignement et de fixation d'os selon la revendication 8, dans lequel le trou latéral est disposé au niveau de l'angle antérieur (26, 126) et s'étend dans un sens sensiblement orthogonal à l'axe longitudinal.

10. Dispositif (10, 110) d'alignement et de fixation d'os selon la revendication 8 ou 9, dans lequel le trou latéral coupe le trou (32, 132) pour outil.

11. Dispositif (10, 110) d'alignement et de fixation d'os selon l'une quelconque des revendications précédentes, dans lequel le trou (32, 132) pour outil s'étend complètement à travers le corps depuis la surface (12, 112) faisant face à l'os jusqu'à la surface opposée (14, 114).

12. Dispositif (10, 110) d'alignement et de fixation d'os selon l'une quelconque des revendications précédentes, comprenant en outre une fente (150) de rotation formée à partir d'une pluralité de trous d'interconnexion s'étendant dans le sens supérieur-inférieur.

13. Trousse (100) pour chirurgie orthopédique d'un os du pied et/ou de la cheville, comprenant :
au moins un dispositif (10, 110) d'alignement et de fixation d'os selon l'une quelconque des revendications précédentes ; et
une pince (38) pour aligner un os déplacé.

14. Trousse (100) selon la revendication 13, comprenant en outre au moins un(e) parmi un fil (40) de guidage, une broche (42), une vis (44) pour os, un guide-fil (46), une jauge (48) de profondeur et/ou un scalpel.

15. Trousse (100) selon la revendication 13 ou 14, dans laquelle l'au moins un dispositif (10, 110) d'alignement et de fixation d'os inclut un premier dispositif d'alignement et de fixation d'os et un deuxième dispositif d'alignement et de fixation d'os, les premier et deuxième dispositifs d'alignement et de fixation d'os étant des images miroir l'un de l'autre.
